# EUROPÄISCHE PATENTSCHRIFT

(11) **EP 0 416 401 B1**
(45) Veröffentlichungstag und Bekanntmachung des Hinweises auf die Patenterteilung: **30.10.1996**
(21) Anmeldenummer: 90116298.2
(22) Anmeldetag: 25.08.1990
(51) Int. Cl.: C07F 9/10, C07F 9/09, A61K 31/685

(54) **Verwendung von Phospholipid-Derivaten als antivirale Arzneimittel und neue Phospholipide**
Use of derivatives of phospholipides as antiviral pharmaceuticals and phospholipides
Utilisation de dérivés de phospholipides comme médicaments et phospholipides

(30) Priorität: 02.09.1989 DE 3929217
(43) Veröffentlichungstag der Anmeldung: 13.03.1991
(73) Patentinhaber: BOEHRINGER MANNHEIM GMBH, 68298 Mannheim (DE)
(72) Erfinder: Herrmann, Dieter, Dr. med., D-6900 Heidelberg (DE); Bosies, Elmar, Dr. phil. nat., D-6940 Weinheim (DE); Zilch, Harald, Dr. rer. nat., D-6800 Mannheim 31 (DE); Koch, Edith, Dr. rer. nat., D-8122 Penzberg (DE)

(56) Entgegenhaltungen:
- EP-A- 0 043 472
- EP-A- 0 050 327
- EP-A- 0 069 968
- EP-A- 0 169 812
- EP-A- 0 395 849
- DE-A- 3 204 735
- DE-A- 3 638 126
- CHEMICAL ABSTRACTS, vol. 112, no. 4, 22. Januar 1990, Columbus, Ohio, US; abstract no. 25650F, Seite 312 ;Spalte 1 ;

## Beschreibung

In der EP-B-0 050 327 werden schwefelhaltige Phospholipide beschrieben, sowie deren Verwendung zur Herstellung von Arzneimitteln. In dieser Patentschrift ist jedoch ausschließlich die cancerostatische Wirkung dieser Phospholipide beschrieben, die sich insbesondere zur Herstellung von Antitumormitteln eignen.

In der EP-A-0 395 849 sind Sulfoxide und Sulfone als ausgewählte Substanzen aus der EP-B-0 050 327 beschrieben, die eine nicht vorhersehbare bessere Wirkung zeigen.

In der DE-A-3 638 126 werden Phosphocholine beschrieben, die in 2-Stellung des C₃-Grundgerüsts den Cholinrest tragen und in 1- und 3-Stellung durch Alkyl/Alkoxy und Alkylmercapto substituiert sind. Diese Verbindungen sind als Therapeutica für durch PAF-aufgelöstes Asthma beschrieben.

In der EP-A-0 043 472 sind 1-S-Alkyl-2-0-acyl-3-phosphochinolin-1-mercapto-2,3-propandiole zur Behandlung von Bluthochdruck und thromboembolischer Erkrankungen beschrieben.

Der JP-A-1 029 319 beschreibt Phospholipide mit antiviraler Wirkung, jedoch sind diese Verbindungen nicht schwefelhaltig.

Aus Gazz. Chem. Ital. 116, 25 (1986) ist die Verbindung 3-Hexadecylmercapto-2-hexadecyloxypropyl-1-phosphorsäure-monocholinester mit potentieil immunstimulierender Wirkung bereits bekannt.

Es wurde nun überraschenderweise gefunden, daß diese Phospholipide eine ausgeprägte antivirale Wirkung aufweisen, und sich daher besonders gut zur Behandlung von viralen bzw. retro-viralen Infektionen eignen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxythymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431).

Es besteht daher ein sehr großes Bedürfnis an Chemotherapeutika, die möglichst spezifisch mit viral oder retroviral bedingten Erkrankungen oder deren Symptomen interferieren, ohne jedoch die anderen, normal ablaufenden natürlichen Körperfunktionen zu beeinflussen.

Der vorliegenden Erfindung lag daher die Aufgabe zugrunde, anti-viral wirksame Mittel und neue Phospholipid-Derivate zur Verfügung zu stellen.

Diese Aufgabe wird durch die in den Ansprüchen gekennzeichneten Merkmale gelöst.

Die Phospholipide der Formel I hemmen sehr spezifisch die Virusvermehrung, wobei insbesondere folgende Viren zu nennen sind: HIV, Herpes, Sendai, Cytomegalie (CMV), Influenza, Parainfluenza, Epstein-Barr (EBV), Vesikular Stomatitis Virus (VSV), Hepatitis, Meningitis, Enzephalitis, etc.. Die genannten Verbindungen können vorteilhaft prophylaktisch oder therapeutisch bei der Behandlung von allen Krankheiten eingesetzt werden, bei denen eine virale bzw. retrovirale Infektion von pathophysiologischer, symptomatischer oder klinischer Relevanz ist.

Gegenstand der vorliegenden Erfindung sind nun neue Verbindungen, die eine Auswahl aus den in der EP-B-0 050 327 beschriebenen schwefelhaltigen Phospholipiden darstellen. Die neuen Verbindungen sind Verbindungen der Formel I. 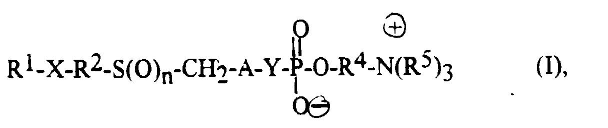 in der
- R¹-X-R²: einen C₈-C₁₆-Alkylrest darstellt,
- n: die Zahlen 0, 1 oder 2 bedeutet,
- R⁴: eine geradkettige oder verzweigte Alkylenkette mit 2 - 4 Kohlenstoffatomen,
- R⁵: Wasserstoff oder eine C₁-C₆-Alkylgruppe ist und die drei Substituenten R⁵ am Stickstoffatom gleich oder verschieden sein können,
- Y: Sauerstoff oder Schwefel ist,
- A: die Gruppe 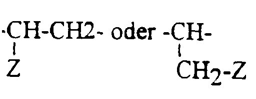 darstellt und
- Z: eine C₁₀-C₁₅-Alkylmercapto-, C₁₀-C₁₅-Alkoxy-, C₁₀-C₁₅-Alkoxycarbonyl-, C₁₀-C₁₅ -Alkylaminocarbonyl- oder C₁₀-C₁₅-Alkylcarbonylaminogruppe bedeutet,
sowie deren pharmakologisch unbedenklichen Salze und optischen Isomere. In der allgemeinen Formel I bedeutet
- R⁴: insbesondere eine geradkettige oder verzweigte C₂-C₄-Alkylengruppe, wobei die Ethylengruppe bevorzugt ist.

Für R⁵ kommt insbesondere ein Wasserstoffatom oder eine C₁-C₄-Alkylgruppe, vorzugsweise die Methylgruppe in Frage.

Unter der Definition der -N⁺(R⁵)₃-Gruppe bei Verbindungen der Formel I sollen sowohl die identisch substituierten Stickstoffderivate, wie z.B. die Trimethylammoniumqmppe, als auch die gemischt substituierten Derivate, wie z.B. Dimethylammonium, Diethyl ammonium-, n-Butyl-dimethylammonium oder Methyl-di-ethylammoniumgruppe verstanden werden bzw. alle durch die Kombination der in der Definition von R⁵ genannten Gruppen möglichen Varianten.

Besonders bevorzugte Verbindungen der allgemeinen Formel I sind solche, in denen die Gruppe R¹-X-R²- eine C₈-C₁₆-Alkylqruppe darstellt, wie beispielsweise die Octyl-, Decyl-, Undecyl-, Dodecyl-, Tridecyl-, Pentadecyl- und Hexadecylgruppe, und Z eine C₁₀-C₁₅-Alkylmercapto-, C₁₀-C₁₅-Alkoxy-, C₁₀-C₁₅-Alkoxycarbonyl-, C₁₀-C₁₅-Alkylaminocarbonyl- oder C₁₀-C₁₅-Alkylcarbonylaminoqrupr wie beispielsweise die Decylmercapto-, Undecylmercapto-, Decyloxy-, undecyloxy-, Dodecyloxy-, Decyloxycarbonyl-, Decylaminocarbonyl- oder Decylcarbonylaminogruppe.

Die Verbindungen der Formel I können nach den in EP-A-50,327 beschriebenen Verfahren hergestellt werden.

Zur Herstellung der Verbindungen der Formel I eignet sich jedoch vorzugsweise das folgende Verfahren: Eine Verbindung der allgemeinen Formel II

R¹-X-R²-S(O)ₙ-CH₂-A-Y^{H} (II)

in der R₁-X-R², n und Y die oben angegebene Bedeutung haben, wird mit einer Verbindung der allgemeinen Formel III 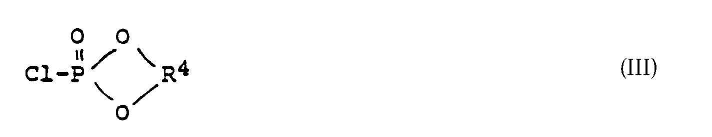 in der R⁴ die oben genannte Bedeutung hat, in Gegenwart eines säurebindenden Mittels umsetzt und das Reaktionsprodukt direkt mit gegebenenfalls alkyliertem Ammoniak behandelt. Die Verbindungen können analog zu LIPIDS 22, 947 (1987) und dort weiter zitierter Literatur hergestellt werden. Verbindungen der Formel III sind kommerziell erhältlich.

Das Verfahren wird in der Regel so durchgeführt, daß ein Alkanol bzw. Alkanthiol der allgemeinen Formel II mit einer Verbindung der allgemeinen Formel III in Gegenwart eines säurebindenden Mittels, wie z.B. Triethylamin in einem absoluten, inerten organischen Lösungsmittel, wie z.B. chlorierter Kohlenwasserstoff oder Toluol, bei Temperaturen um den Gefrierpunkt bis Raumtemperatur reagiert, und das Reaktionsprodukt direkt mit gegebenenfalls alkyliertem Ammoniak behandelt wird. Dazu löst man den Ammoniak oder das Alkylamin in einem Medium, das sowohl den Phosphorsäurediester als auch Ammoniak oder das Amin genügend gut löst, wozu sich besonders Mischungen aus Acetonitril oder niederen Alkoholen mit chlorierten Kohlenwasserstoffen eignen und vervollständigt die Reaktion bei einer Temperatur von 20 bis 70°C.

Man kann auch stufenweise vorgehen, also zuerst eine Alkylammoniumgruppe einführen und mit Alkylhalogenid anschließend zum Di- oder Trialkylammoniumalkylester umsetzen.

Sämtliche Zwischenstufen sowie Endprodukte lassen sich bequem säulenchromatographisch mit üblichen Elutionsmitteln, wie z.B. Ether, Ligroin, chlorierten Kohlenwasserstoffen, niederen Alkoholen oder Mischungen derselben, an Kieselgel reinigen, wobei im Falle des betainartigen Endprodukts zweckmässig etwas Wasser zugesetzt wird.

Die Verbindungen der Formel I können auch nachträglich in andere Verbindungen der Formel I umgewandelt werden, wie z.B. durch Oxidation von Verbindungen mit n=0 in Verbindungen mit n=1 oder n=2.

Die pharmakologisch verträglichen Salze erhält man in üblicher Weise, z.B. durch Neutralisation der Verbindungen der Formel I mit nichttoxischen anorganischen oder organischen Säuren, wie z.B. Salz-, Schwefel-, Phosphor-, Bromwasserstoff-, Essig-, Milch-, Zitronen-, Äpfel-, Salicyl-, Malon-, Malein- oder Bernsteinsäure.

Die Arzneimittel enthaltend Verbindungen der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragées, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylendiamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage :
1. 3-Dodecylmercapto-2-decylmercaptopropyl-1-phosphorsäure-mono-cholinester
2. 3-Dodecylmercapto-2-decyloxycarbonylpropyl-1-phosphorsäure-monocholinester
3. 3-Dodecylmercapto-2-decylaminocarbonylpropyl-1-phosphorsäure-monocholinester
4. 3-Undecylsulfonyl-2-undecyloxypropyl-1-phosphorsäure-monocholinester
5. 3-Tridecylsulfinyl-2-undecyloxypropyl-1-phosphorsäure-monocholinester
6. 3-Tridecylsulfonyl-2-undecyloxypropyl-1-phosphorsäure-monocholinester
7. 3-Tetradecylsulfonyl-2-undecyloxypropyl-1-phosphorsäure-monocholinester
8. 3-Tridecylmercapto-2-decylmercaptopropyl-1-phosphorsäure-monocholinester
9. 3-Tetradecylmercapto-2-decylmercaptopropyl-1-phosphorsäure-monocholinester
10. 3-Decylmercapto-1-dodecyloxypropyl-2-phosphorsäure-monocholinester
11. 3-Dodecylmercapto-1-decyloxypropyl-2-phosphorsäure-monocholinester
12. 1,3-Bis-(undecylmercapto)-propyl-2-phosphorsäure-monocholinester

### Beispiel 1

### 3-Dodecylmercapto-2-decyloxypropyl-1-phosphorsäure-monocholinester

4.4 g 3-Dodecylmercapto-2-decyloxypropanol-1 in 30 ml Dichlormethan sowie 4.1 ml Triethylamin werden bei -25°C mit einer Lösung aus 2.6 g 2-Chlor-2-oxo-1,3,2-dioxaphospholan (Fluka) in 10 ml Dichlormethan versetzt und 30 Minuten gerührt. Dann läßt man auf RT erwärmen, entfernt das Lösungsmittel im Rotationsverdampfer und suspendiert den Rückstand in Ether. Nach Abtrennen das Triethylammoniumsalzes wird das Filtrat erneut eingedampft und der ölige Rückstand in 30 ml Acetonitril aufgenommen. Die klare Lösung wird durch Einleiten bei 20°C mit Trimethylamin gesättigt und 96 Stunden bei RT im geschlossenen Gefäß gerührt. Danach wird der Niederschlag abgesaugt, mit Acetonitril gewaschen und aus Dichlormethan/Aceton umkristallisert. Ausb. 4.48 g (58 % d.Th.), Schmp. 196-200°C.

Das Ausgangsmaterial wurde analog zu LIPIDS 22, 947 (1987) und dort zitierter Literatur hergestellt, indem man das Natriumsalz des 1,3-Benzylidenglyzerins mit Decylbromid in DMF umsetzt (Chromatographie an Kieselgel mit Ether/Hexan 1/5 als Eluens, gelbes öl, Ausb. 59 %), das erhaltene 1,3-Dioxan mit NBS in Dichlormethan öffnet zum 3-Brom-2-decyloxypropylbenzoat (gelbes öl, als Rohprodukt in die nächste Reaktion eingesetzt) und dieses mit dem Natriumsalz des Dodecylmercaptans in Ethanol zur Reaktion bringt (Chromatographie an Kieselgel mit Ether/Hexan 1/5 als Eluens, gelbes Öl, Ausb. 78 %).

### Beispiel 2

3-Decylmercapto-2-dodecyloxypronyl-1-phosphorsäure-monocholinester wurde analog zu Bsp. 1 hergestellt.
Ausbeute 44 %, Schmp. 195°C.
Das als Edukt verwendete 3-Decylmercapto-2-dodecyloxypropanol-1 wurde wie unter Bsp. 1 beschrieben hergestellt (farbloses öl, Ausb. 61 %).

### Beispiel 3

3-Decylmercapto-2-decyloxypropyl-1-phosphorsäure-monocholinester wurde analog zu Bsp. 1 hergestellt.
Ausbeute 37 %, wachsartige Konsistenz.
Das als Edukt verwendete 3-Decylmercapto-2-decyloxynropanol-1 wurde wie unter Bsp. 1 beschrieben hergestellt (gelbliches Öl, Ausb. 47 %).

### Beispiel 4

3-Dodecylmercapto-2-dodecyloxypropyl-1-phosphorsäure-monocholinester wurde analog zu Bsp. 1 hergestellt.
Ausbeute 61% %, Schmp. 230°C (Zers.).
Das Ausgangsmaterial 3-Dodecylmercapto-2-dodecyloxypropanol-1 erhielt man analog zum unter Bsp. 1 beschriebenen Verfahren (gelbliches Öl, Ausb. 42 %).

### Beispiel 5

3-Hexadecylmercapto-2-decyloxypropyl-1-phosphorsäure-monocholinester wurde analog zu Bsp. 1 hergestellt.
Ausbeute 47 %, Schmp. 192°C.
Das als Edukt verwendete 3-Hexadecylmercapto-2-decyloxypropanol-1 erhielt man analog zum unter Bsp. 1 beschriebenen Verfahren (gelbes Öl, Ausb. 57 %).

### Beispiel 6

3-Decylmercapto-2-hexadecyloxypropyl-1-phosphorsäure-monocholinester wurde analog zu Bsp. 1 hergestellt.
Ausbeute 59 %, Schmp. 232°C (Zers.).
Das Ausgangsmaterial 3-Decylmercapto-2-hexadecyloxypropanol-1 wurde wie unter Bsp. 1 beschrieben hergestellt (gelbes Öl, Ausb. 55 %).

### Beispiel 7

3-Hexadecylmercapto-2-hexadecyloxypropyl-1-phosphorsäure-monocholinester wurde analog zu Bsp. 1 hergestellt.
Ausbeute 59 %, Schmp. 233°C (Zers.).
Die Verbindung ist auf einem anderen Syntheseweg bereits in Gazz. Chim. Ital. 116, 25 (1986) beschrieben.
Das als Edukt verwendete 3-Hexadecylmercapto-2-hexadecyloxypropanol-1 wurde wie unter Bsp. 1 beschrieben hergestellt (gelbliches Öl, Ausb. 47 %).

### Beispiel 8

### 3-Dodecylsulfonyl-2-decyloxypropyl-1-phosphorsäure-monocholinester

1 g 3-Dodecylmercapto-2-decyloxypropyl-1-phosphorsäure-monocholinester (Bsp. 1) wird in 10 ml Eisessig gelöst, mit 1.8 ml 30 % H₂O₂ versetzt und 20 Stunden bei 50°C gerührt. Danach entfernt man das Lösungsmittel im Rotationsverdampfer, versetzt mit Wasser, engt erneut ein und versetzt mit Aceton. Nach Stehen über Nacht im Kühlschrank werden die Kristalle abgesaugt, mit Aceton und Ether gewaschen und getrocknet.
Ausbeute 750 mg (71 % d.Th.), Schmp. 223-230°C.

### Beispiel 9

3-Decylsulfonyl-2-dodecyloxypropyl-1-phosphorsäure-monocholinester wurde analog zu Bsp. 8 aus dem 3-Decylmercapto-2-dodecyloxypropyl-1-phosphorsäure-monocholinester(Bsp. 2) hergestellt. Ausbeute 66 % d.Th., Schmp. 223-227°C.

### Beispiel 10

3-Undecylmercapto-2-undecyloxypropyl-1-phosphorsäure-monocholinester wurde analog zu Bsp. 1 hergestellt.
Ausbeute 71 %, Schmp. 230-233°C (Zers.).

Das als Ausgangsmaterial verwendete 3-Undecylmercapto-2-undecyloxypropanol-1 wurde ebenfalls wie unter Bsp. 1 beschrieben hergestellt (Öl, Ausbeute 49 %).

### Beispiel 11

3-Undecylmercapto-2-decyloxypropyl-1-phosphorsäuremonocholinester wurde analog zu Beispiel 10 hergestellt. Ausbeute 64 %, Schmp. 227-231° C (Zers.).

### Beispiel 12

3-Tridecylmercapto-2-decyloxypropyl-1-phosphorsäuremonocholinester wurde analog zu Beispiel 10 hergestellt. Ausbeute 55 %, Schmp. 229-232° C (Zers.).

### Beispiel 13

3-Tetradecylmercapto-2-decyloxypropyl-1-phosphorsäuremonocholinester wurde analog zu Beispiel 10 hergestellt. Ausbeute 63 %, Schmp. 209-214° C.

### Beispiel 14

3-Pentadecylmercapto-2-decyloxypropyl-1-phosphorsäure-monocholinester wurde analog zu Beispiel 10 hergestellt. Ausbeute 31 %, Schmp. 185° C.

### Beispiel 15

3-Octylmercapto-2-decyloxypropyl-1-phosphorsäuremonocholinester wurde analog zu Beispiel 10 hergestellt. Ausbeute 45 %, Schmp. 223-228° C.

### Beispiel 16

3-Decylmercapto-2-octyloxypropyl-1-phorphorsäuremonolinester wurde analog zu Beispiel 10 hergestellt. Ausbeute 35 %, Schmp. 221-225° C.

### Beispiel 17

3-Dodecylmercapto-2-octyloxypropyl-1-phosphorsäuremonocholinester wurde analog zu Beispiel 10 hergestellt. Ausbeute 43 %, Schmp. 198-206° C.

### Beispiel 18

3-Dodecylmercapto-2-methoxypropyl-1-phosphorsäure-monocholinester wurde analog zu Beispiel 10 hergestellt. Ausbeute 39 %, Schmp. >270° C (Zers.).

### Beispiel 19

2,3-Bis-(undecylmercapto)-propyl-1-phosphorsäure-monocholinester wurde analog zu Beispiel 10 hergestellt. Ausbeute 62%, Schmp. 197-205° C. Die Ausgangsverbindung 2,3-Bis-(undecylmercapto)propanol-1 wird durch Umsetzung von 2,3-Dibrompropan-1-ol mit dem Natriumsalz des Undecylmercaptans in DMF in 61 % Ausbeute als Öl erhalten.

### Beispiel 20

1,3-Bis-(dodecylmercapto)-propyl-2-phosphorsäure-monocholinester wurde analog zu Beispiel 10 hergestellt. Ausbeute 26 %, Schmp. 208-212° C. Die Ausgangsverbindung 1,3-Bis-(dodecylmercapto)-propan-2-ol wird durch Umsetzung von 1,3-Dibrompropan-2-ol mit dem Natriumsalz des Dodecylmercaptans in DMF in 67 % Ausbeute als Öl erhalten.

### Beispiel 21

### 3-Dodecylmercapto-2-decylcarbonylamino-propyl-1-phosphorsäure-monocholinester

a) 3-Dodecylmercapto-2-aminopropionsäure
   16.5 g (0.42 mol) festes Natriumhydroxid wurden in 1600 ml Ethanol gelöst, mit einer Lösung aus 25 g (0.21 mol) D,L-Cystein in 150 ml Ethanol versetzt und nach Zutropfen von 60 ml (0.25 mol) Dodecylbromid 24 h bei Raumtemperatur gerührt. Dann wurde der Niederschlag abgesaugt, mit Ethanol gewaschen und in Wasser gelöst. Nach Ansäuern mit 4 N Salzsäure ließ sich der Niederschlag nach 2 h bei 5°C absaugen. Ausbeute 48.2 g (81 %), Schmp. 215-218°C (Zers.).
b) 3-Dodecylmercapto-2-aminopropionsäureethylester
   Die freie Säure der letzten Reaktion wurde in 1 l Ethanol suspendiert und unter gelindem Einleiten von Chlorwasserstoff 4 h unter Rückfluß erhitzt. Nach dem Abkühlen wurde das Lösungsmittel entfernt und das Hydrochlorid aus Ether/Isohexan kristallisiert. Ausbeute 48.5 g (84 %), Schmp. 103-106°C.
c) 3-Dodecylmercapto-2-decylcarbonylaminopropionsäure-ethylester
   8.7 g (24.6 mol) des Hydrochlorids der letzten Reaktion wurden in einer Mischung aus 90 ml Dichlormethan und 10.5 ml Triethylamin suspendiert und bei RT langsam mit 5.6 g (27.4 mmol) Undecanoylchlorid in 50 ml Dichlormethan versetzt. Die Suspension wurde über Nacht gerührt, mit 200 ml ges. NH₄Cl-Lösung versetzt, die organische Phase mit Wasser gewaschen, getrocknet und eingedampft. Der Rückstand wurde ohne weitere Reinigung in die nächste Reaktion eingesetzt.
d) 3-Dodecylmercapto-2-decylcarbonylamino-propanol-1
   Das Rohprodukt der letzten Reaktion in 25 ml THF wurde bei RT zu einer Suspension aus 0.9 g LiBH₄ in 25 ml THF getropft und 3 h nachgerührt. Dann wurde vorsichtig durch Zugabe von 50 ml ges. NH₄Cl-Lösung hydrolysiert, mit 100 ml Ether verdünnt, die organische Phase nach dem Ausschütteln abgetrennt, getrocknet und eingedampft. Der Rückstand wurde durch Chromatographie an Kieselgel mit Ether/Isohexan als Eluens gereinigt. Ausbeute 8.9 g (81 %, bezogen auf den eingesetzten 3-Dodecylmercapto-2-aminopropionsäureethylester), Schmp. 64-67°C.
e) 3-Dodecylmercapto-2-decylcarbonylamino-propan-1-phosphorsäure-monocholinester wurde in Analogie zu Bsp. 1 hergestellt.
   Ausbeute 67 %, Schmp. 191-194°C.

### Beispiel 22

### 3-Dodecylsulfinyl-2-decyloxypropyl-1-phosphorsäure-monocholinester

1 g 3-Dodecylmercapto-2-decyloxypropyl-1-phosphorsäure-monocholinester (Bsp. 1) wurde in 8 ml Eisessig gelöst, mit 0.2 ml 30 % H₂O₂ versetzt und 3 Tage bei RT gerührt. Danach wurde im Rotationsverdampfer eingeengt und der Rückstand aus Aceton kristallisiert. Ausbeute 0.65 g (63 %), Schmp. 230-233°C (Zers.)

### Beispiel 23

3-Tetradecylsulfinyl-2-decyloxypropyl-1-phosphorsäure-monocholinester wurde analog zu Beispiel 22 aus dem 3-Tetradecylmercapto-Derivat (Bsp. 13) in 71 % Ausbeute, Schmp. 225-230°C hergestellt.

### Beispiel 24

3-Undecylsulfinyl-2-undecyloxypropyl-1-phosphorsäure-monocholinester wurde analog zu Beispiel 22 aus dem 3-Undecylmercapto-Derivat (Bsp. 10) in 61 % Ausbeute, Schmp. 232-235°C (Zers.) hergestellt.

### Beispiel 25

3-Tridecylsulfinyl-2-decyloxypropyl-1-phosphorsäure-monocholinester wurde analog zu Beispiel 22 aus dem 3-Tridecylmercapto-Derivat (Bsp. 12) in 68 % Ausbeute, Schmp. 232-234°C (Zers.) hergestellt.

### Beispiel 26

3-Decylsufinyl-2-decyloxypropyl-1-phosphorsäure-monocholinester wurde analog zu Beispiel 22 aus dem 3-Decylmercapto-Derivat (Bsp. 2) in 61 % Ausbeute, Schmp. >226°C (Zers.) hergestellt.

### Beispiel 27

3-Undecylmercapto-2-decyloxymethylpropyl-1-phosphorsäure-monocholinester wurde analog zu LIPIDS, 22, 947 (1987) hergestellt, wobei das 5-Decyloxymethyl-2-phenyl-1,3-dioxan durch Reaktion des 5-Hydroxymethyl-Derivats mit Natriumhydrid in DMF und Umsetzung mit Decylbromid in 73 % Ausbeute erhalten wurde. Ausbeute 49 %. Schmp. 217-220°C (Zers.).

### Beispiel 28

1-Dodecylmercapto-3-decyloxypropyl-2-phosphorsäure-monocholinester
a) 1-Decyloxy-2,3-epoxypropan
   4.23 ml (0.05 mol) Epibromhydrin in einem Zweiphasengemisch aus 150 ml Dichlormethan, 150 ml 50 % NaOH und 3.4 g Tetrabutylammoniumhydrogensulfat wurden bei RT mit 9.52 ml (0.05 mol) 1-Decanol versetzt und über Nacht gerührt. Dann wurde mit Wasser verdünnt, die organische Phase abgetrennt, mit Wasser gewaschen und eingedampft. Der ölige Rückstand wurde durch Säulenchromatographie an Kieselgel 60 mit Ether/Isohexan 1/15 gereinigt. Ausbeute 5.0 g (47 %).
b) 1-Dodecylmercapto-3-decyloxypropanol-2
   1.6 g (7.5 mmol) des Epoxids der letzten Reaktion in 16 ml Ethanol wurden bei RT mit 2.3 ml (9.6 mmol) Dodecylmercaptan und 0.2 g gepulverter KOH versetzt und über Nacht gerührt. Danach wurde mit Wasser verdünnt, mit Dichlormethan extrahiert, die organische Phase mit Wasser gewaschen, getrocknet und eingedampft. Der Rückstand wurde durch Säulenchromatographie an Kieselgel 60 mit Ether/Isohexan 1/5 gereinigt. Ausbeute 2.4 g (76 %), farbloses Öl.
c) 1-Dodecylmercapto-3-decyloxypropyl-2-phosphorsäure-monocholinester wurde analog zu Bsp. 1 hergestellt. Ausbeute 54 %, Schmp. 219-224°C.

## Patentansprüche

1. Verbindungen der Formel I 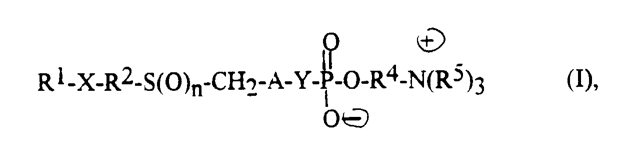 in der
R¹-X-R² einen C₈-C₁₆-Alkylrest darstellt,
n die Zahlen 0, 1 oder 2 bedeutet,
R⁴ eine geradkettige oder verzweigte Alkylenkette mit 2 - 4 Kohlenstoffatomen,
R⁵ Wasserstoff oder eine C₁-C₆-Alkylgruppe ist und die drei Substituenten R⁵ am Stickstoffatom gleich oder verschieden sein können,
Y Sauerstoff oder Schwefel ist,
A die Gruppe 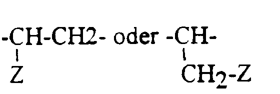 darstellt und
Z eine C₁₀-C₁₅-Alkylmercapto-, C₁₀-C₁₅-Alkoxy-, C₁₀-C₁₅-Alkoxycarbonyl-, C₁₀-C₁₅-Alkylaminocarbonyl- oder C₁₀-C₁₅-Alkylcarbonylaminogruppe bedeutet,
sowie deren pharmakologisch unbedenklichen Salze und optischen Isomere.

2. Verbindungen gemäß Anspruch 1, ausgewählt aus der Gruppe der folgenden Verbindungen:
3-Dodecylmercapto-2-decyloxypropyl-1-phosphorsäure-nonocholinester
3-Decylmercapto-2-dodecyloxypropyl-1-phosphorsäure-monocholinester
3-Dodecylmercapto-2-dodecyloxypropyl-1-phosphorsäure-monocholinester
3-Undecylmercapto-2-undecyloxypropyl-1-phosphorsäure-monocholinester
3-Tridecylmercapto-2-decyloxypropyl-1-phosphorsäure-nonocholinester
3-Dodecylmercapto-2-decylmercaptopropyl-1-phosphorsäure-monocholinester
3-Dodecylsulfonyl-2-decyloxypropy1-1-phosphorsäure-nonocholinester
3-Decylsulfonyl-2-dodecyloxypropyl-1-phosphorsäure-nonocholinester
2-Undecylsulfinyl-2-undecyloxypropyl-1-phosphorsäure-monocholinester.

3. Arzneimittel enthaltend mindestens eine Verbindung gemäß Anspruch 1 oder 2 neben üblichen Träger- und Hilfsstoffen.

4. Verwendung von Verbindungen gemäß Anspruch 1 oder 2 zur Herstellung von Arzneimitteln mit antiviraler Wirkung.

5. Verfahren zur Herstellung von Verbindungen der Formel I gemäß einem der Ansprüche 1 oder 2, dadurch gekennzeichnet, daß man eine Verbindung der Formel II
R¹-X-R²-S(O)ₙ-CH₂-A-YH (II),
in der
R¹-X-R² einen C₈-C₁₆-Alkylrest darstellt,
n die Zahlen 0, 1 oder 2 bedeutet,
Y Sauerstoff oder Schwefel ist,
A die Gruppe 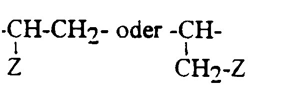 darstellt und
Z eine C₁₀-C₁₅-Alkylmercapto-, C₁₀-C₁₅-Alkoxy-, C₁₀-C₁₅-Alkoxycarbonyl-, C₁₀-C₁₅-Alkylaminocarbonyl- oder C₁₀-C₁₅-Alkylcarbonylaminogruppe bedeutet,
mit einer Verbindung der allgemeinen Formel III 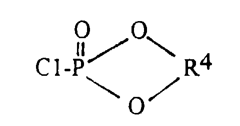 in der
R⁴ eine geradkettige oder verzweigte Alkylenkette mit 2 - 4 Kohlenstoffatomen ist,
in Gegenwart eines säurebindenden Mittels umsetzt und das Reaktionsprodukt direkt mit gegebenenfalls alkyliertem Ammoniak behandelt und anschließend isoliert.

6. Verfahren zur Herstellung von Arzneimitteln enthaltend mindestens eine Verbindung der Formel I gemäß einem der Ansprüche 1 oder 2, dadurch gekennzeichnet, daß man Verbindungen der Formel I mit pharmakologisch üblichen Hilfs- oder Trägerstoffen vermischt und zu pharmazeutischen Zubereitungen verarbeitet.

## Claims

1. Compounds of the formula I 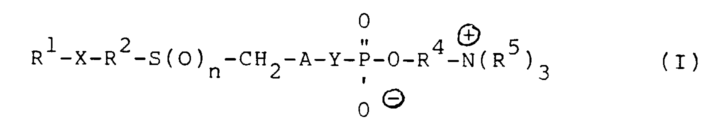 in which R¹-X-R² represents a C₈-C₁₆-alkyl radical, n signifies the numbers 0, 1 or 2, R⁴ a straight-chained or branched alkylene chain with 2 - 4 carbon atoms, R⁵ is hydrogen or a C₁-C₆-alkyl group and the three substituents R⁵ on the nitrogen atom can be the same or different, Y is oxygen or sulphur, A represents the group 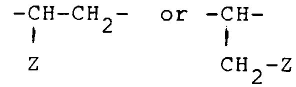 and Z signifies a C₁₀-C₁₅-alkylmercapto, C₁₀-C₁₅-alkoxy, C₁₀-C₁₅-alkoxycarbonyl, C₁₀-C₁₅-alkylaminocarbonyl or C₁₀-C₁₅-alkylcarbonylamino group, as well as their pharmacologically compatible salts and optical isomers.

2. Compounds according to claim 1 selected from the group of the following compounds:
3-dodecylmercapto-2-decyloxypropyl-1-phosphoric acid monocholine ester,
3-decylmercapto-2-dodecyloxypropyl-1-phosphoric acid monocholine ester,
3-dodecylmercapto-2-dodecyloxypropyl-1-phosphoric acid monocholine ester,
3-undecylmercapto-2-undecyloxypropyl-1-phosphoric acid monocholine ester,
3-tridecylmercapto-2-decyloxypropyl-1-phosphoric acid monocholine ester,
3-dodecylmercapto-2-decylmercaptopropyl-1-phosphoric acid monocholine ester,
3-dodecylsulphonyl-2-decyloxypropyl-1-phosphoric acid monocholine ester,
3-decylsulphonyl-2-dodecyloxypropyl-1-phosphoric acid monocholine ester,
3-undecylsulphinyl-2-undecyloxypropyl-1-phosphoric acid monocholine ester.

3. Medicament containing at least one compound according to claim 1 or 2, besides usual carrier and adjuvant materials.

4. Use of compounds according to claim 1 or 2 for the preparation of medicaments with antiviral action.

5. Process for the preparation of compounds of the formula I according to one of claims 1 or 2, characterised in that one reacts a compound of the formula II
R¹-X-R²-S(O)ₙ-CH₂-A-YH (II)
in which R¹-X-R² represents a C₈-C₁₆-alkyl radical,
n signifies the numbers 0, 1 or 2, Y is oxygen or sulphur,
A represents the group 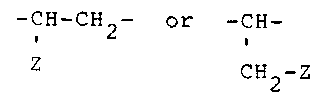 and Z signifies a C₁₀-C₁₅-alkylmercapto, C₁₀-C₁₅-alkoxy, C₁₀-C₁₅-alkoxycarbonyl, C₁₀-C₁₅-alkylaminocarbonyl or C₁₀-C₁₅-alkylcarbonylamino group, with a compound of the general formula III 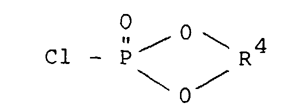 in which R⁴ is a straight-chained or branched alkylene chain with 2 - 4 carbon atoms, in the presence of an acid-binding agent and treats the reaction product directly with possibly alkylated ammonia and subsequently isolates.

6. Process for the production of medicaments containing at least one compound of the formula I according to one of claims 1 or 2, characterised in that one mixes compounds of the formula I with pharmacologically conventional adjuvant and carrier materials and works up to pharmaceutical compositions.

## Revendications

1. Composés de formule I 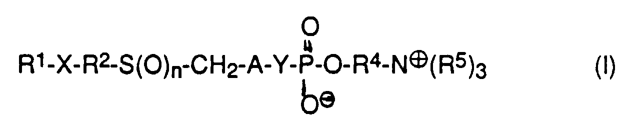 dans laquelle
R¹-X-R² représente un reste alkyle en C₈-C₁₆,
n vaut 0, 1 ou 2,
R⁴ représente une chaîne alkylène linéaire ou ramifiée, ayant de 2 à 4 atomes de carbone,
R⁵ représente un atome d'hydrogène ou un groupe alkyle en C₁-C₆, et les trois substituants R⁵ de l'atome d'azote peuvent être identiques ou différents,
Y représente un atome d'oxygène ou de soufre,
A représente le groupe 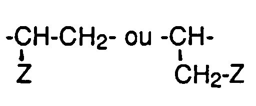 et
Z représente un groupe alkylmercapto en C₁₀-C₁₅, alcoxy en C₁₀-C₁₅, alcoxycarbonyle en C₁₀-C₁₅, alkylaminocarbonyle en C₁₀-C₁₅ ou alkylcarbonylamino en C₁₀-C₁₅,
ainsi que leurs sels pharmacologiquement acceptables et leurs isomères optiques.

2. Composés selon la revendication 1, choisis dans le groupe constitué par les composés suivants :
ester de monocholine de l'acide 3-dodécylmercapto-2-décyloxypropyl-1-phosphorique
ester de monocholine de l'acide 3-décylmercapto-2-dodécyloxypropyl-1-phosphorique
ester de monocholine de l'acide 3-dodécylmercapto-2-dodécyloxypropyl-1-phosphorique
ester de monocholine de l'acide 3-undécylmercapto-2-undécyloxypropyl-1-phosphorique
ester de monocholine de l'acide 3-tridécylmercapto-2-décyloxypropyl-1-phosphorique
ester de monocholine de l'acide 3-dodécylmercapto-2-décylmercaptopropyl-1-phosphorique
ester de monocholine de l'acide 3-dodécylsulfonyl-2-décyloxypropyl-1-phosphorique
ester de monocholine de l'acide 3-décylsulfonyl-2-dodécyloxypropyl-1-phosphorique
ester de monocholine de l'acide 3-undécylsulfinyl-2-undécyloxypropyl-1-phosphorique.

3. Médicament contenant au moins un composé selon la revendication 1 ou 2, en plus de véhicules et adjuvants usuels.

4. Utilisation de composés selon la revendication 1 ou 2, pour la préparation de médicaments ayant une activité antivirale.

5. Procédé de préparation de composés de formule I selon la revendication 1 ou 2, caractérisé par le fait que l'on fait réagir un composé de formule II
R¹-X-R²-S(O)ₙ-CH₂-A-YH (II)
dans laquelle
R¹-X-R² représente un reste alkyle en C₈-C₁₆,
n vaut 0, 1 ou 2,
Y représente un atome d'oxygène ou de soufre,
A représente le groupe 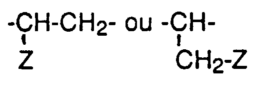 et
Z représente un groupe alkylmercapto en C₁₀-C₁₅, alcoxy en C₁₀-C₁₅, alcoxycarbonyle en C₁₀-C₁₅, alkylaminocarbonyle en C₁₀-C₁₅ ou alkylcarbonylamino en C₁₀-C₁₅,
avec un composé de formule générale III 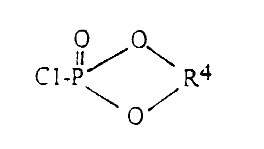 dans laquelle
R⁴ représente une chaîne alkylène linéaire ou ramifiée, ayant de 2 à 4 atomes de carbone,
en présence d'un agent fixant les acides, et on traite le produit réactionnel directement avec de l'ammoniac éventuellement alkylé et ensuite, on isole le produit obtenu.

6. Procédé de préparation de médicaments contenant au moins un composé de formule I, selon la revendication 1 ou 2, caractérisé en ce que l'on mélange des composés de formule I avec des adjuvants ou véhicules pharmacologiques usuels et que l'on transforme le tout en préparations pharmaceutiques.
